(19) Europäisches Patentamt
European Patent Office
Office européen des brevets

(11) EP 0 453 797 B1

## (12) EUROPEAN PATENT SPECIFICATION

(45) Date of publication and mention
of the grant of the patent:
**26.06.1996 Bulletin 1996/26**

(51) Int Cl.⁶: **G01N 21/86**, G01N 33/34

(21) Application number: **91104799.1**

(22) Date of filing: **26.03.1991**

(54) **Infrared ray moisture meter**

Infrarotfeuchtigkeitsmessgerät

Dispositif de mesure de l'humidité par rayons infrarouges

(84) Designated Contracting States:
**DE GB**

(30) Priority: **26.04.1990 JP 111539/90**
**18.01.1991 JP 4544/91**

(43) Date of publication of application:
**30.10.1991 Bulletin 1991/44**

(73) Proprietor: **Yokogawa Electric Corporation**
**Tokyo 180 (JP)**

(72) Inventors:
• **Chiba, Ryuji**
**Funabashi,shi, Chiba 274 (JP)**
• **Hara, Hitoshi**
**Tanashi-shi, Tokyo 188 (JP)**

• **Yamada, Tomoyuki**
**Toshima-ku, Tokyo 171 (JP)**
• **Isozaki, Kenji**
**Mitaka-shi, Tokyo 181 (JP)**

(74) Representative: **Henkel, Feiler, Hänzel & Partner**
**Möhlstrasse 37**
**81675 München (DE)**

(56) References cited:
**EP-A- 0 332 018**      **US-A- 4 027 161**
**US-A- 4 052 615**

• **PATENT ABSTRACTS OF JAPAN, Vol. 13, No.**
**215, (P.874)(3563), 19 May 1989; & JP-A-1032153**
• **PATENT ABSTRACTS OF JAPAN, Vol. 13, No.**
**209 (P-871)(3557), 17 May 1989; & JP-A-1025038**

## Description

BACKGROUND OF THE INVENTION

1. Field of the Invention

The present invention relates to a device for measuring characteristics of paper as defined by the features of the preamble of claim 1 and to an infrared ray moisture meter.

Description of the Prior Art

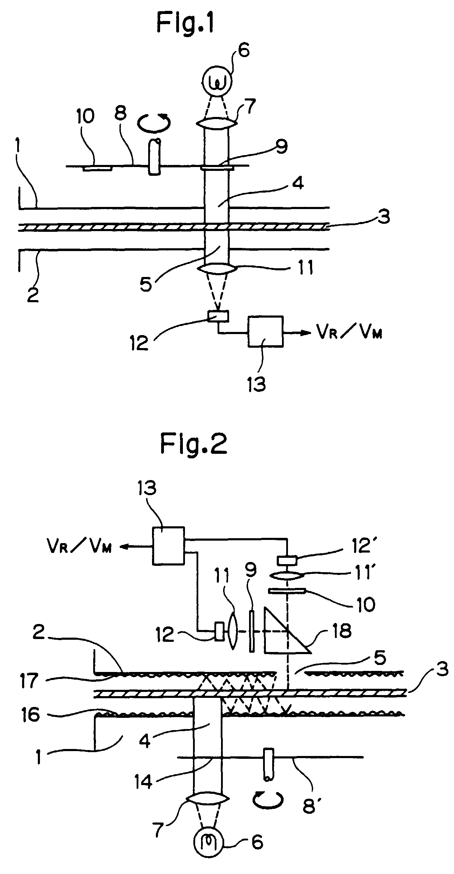

Figs. 1 through 3 show conventional moisture meters which measure moisture of paper in a paper machine, etc.

In Fig. 1, a light emitting portion 1 and a light detecting portion 2 are disposed so as to face each other with paper 3, which is an object to be measured, sandwiched between them.

At the light emitting portion 1, rays from a light source 6 are made to be parallel rays by a lens 7, are further made to be intermittent rays by a chopper wheel 8, and are applied to the paper 3 through an irradiation window 4. The chopper wheel 8 is provided with a filter 9 which transmits rays the wavelength of which rays is 1.94μm and which rays are absorbed by moisture (M rays) and a filter 10 which transmits rays the wavelength of which rays is 1.8μm and which rays are not absorbed by moisture (R rays). According to rotation of the chopper wheel 8, the chopper wheel 8 applies to the paper 3 the M rays and the R rays by turns. At the light detection portion 2, the rays which penetrate the paper 3 are introduced from an incidence window 5, focused by a lens 11, and focused on a light detector 12. At the light detector 12, the M rays and the R rays are detected in time sequence and given to a computing unit 13. Then, computing of $V_R/V_M$ is conducted and the result is outputted.

In a conventional moisture meter known from JP62-189269 which is shown in Fig. 2, at the light emitting portion 1, rays from the light source 6 are made to be parallel rays by the lens 7, are made to be intermittent rays by a chopper wheel 8', and are applied to the paper 3 through the irradiation window 4. Filters such as mounted on the conventional moisture meter which is shown in Fig. 1 are not mounted on the chopper wheel 8' and the chopper wheel 8' is used solely for the purpose of getting rid of the influence of stray light. White light which is applied from the irradiation window 4 is under multiple reflection at irregular reflection surfaces 16 and 17 which are provided on the surface of the light emitting portion 1 and on the surface of the light detecting portion 2, respectively, which face each other with the paper 3 sandwiched between them. Then, the white light is introduced in the light detecting portion 2 from the incidence window 5 which incidence window 5 is provided so as to be misaligned with respect to the irradiation window 4.

At the light detecting portion 2, the introduced light is divided in two by a beam splitter 18. One group of the divided light is introduced to the light detector 12 through the filter 9 which transmits the M rays and through the lens 11. The other group of the divided light is introduced to a light detector 12' through the filter 10 which transmits the R rays and through a lens 11'. The M rays which are detected by the light detector 12 and the R rays which are detected by the light detector 12' are given to the computing unit 13 at the same time. Then, computing of $V_R/V_M$ is conducted and the result is outputted.

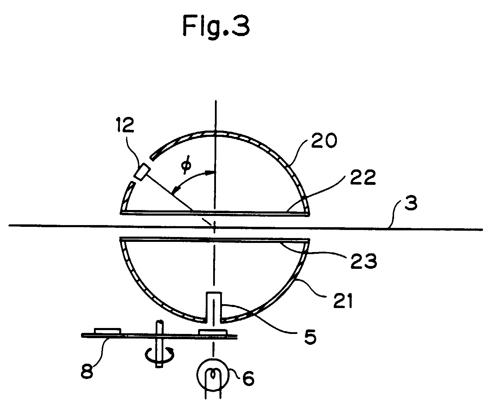

Fig. 3 shows still another conventional moisture meter. Spherical mirrors 20 and 21 are disposed with the openings covered by dustproof glass 22 and 23 and with the paper 3 sandwiched between them. In this moisture meter, rays which are applied by the light source 6 and which are made to be intermittent rays by the chopper wheel 8 having the two kinds of filters that are referred to above are applied to the paper 3 through an irradiation window 5. Then, the rays which penetrate the paper 3 or which are scattered by the paper 3 reach the light detector 12 after penetrating the paper 3 a plurality of times by being reflected at the inner surface of the sphere and by being applied to the paper 3 again. The computing of $V_R/V_M$ is conducted in the same way as in the examples which are shown in Figs. 1 and 2 by a computing unit (not shown) using the detected rays and an electric signal which is related to the moisture of the paper 3 is outputted.

In the conventional infrared ray moisture meters which are referred to above, M rays and R rays are applied to paper, and the ratio of the output $V_R$ of the R rays which penetrate the paper to the output $V_M$ of the M rays which penetrate the paper, that is, $V_R/V_M$ is computed and an electric signal which is related to the moisture weight of the paper is obtained.

In an on-line measurement, as the relationship between the moisture weight and the output of the moisture meter subtly changes depending on the kind of the pulp which is the raw material of the paper and the basis weight of the paper, calibration curves are made using samples which are prepared in advance, the calibration curves are inputted to the computer, a calibration curve the characteristics of which are the nearest to the kind and the basis weight of the paper to be manufactured is selected, and the moisture weight of the paper is obtained using the calibration curve.

However, the number of calibration curves which can be inputted to the computer is limited (for example, eight) and all objects to be measured can not be covered. In a conventional moisture meter, even with respect to paper made from pulp of the same kind, if the basis weight is different, the gap between the calibration curves is big.

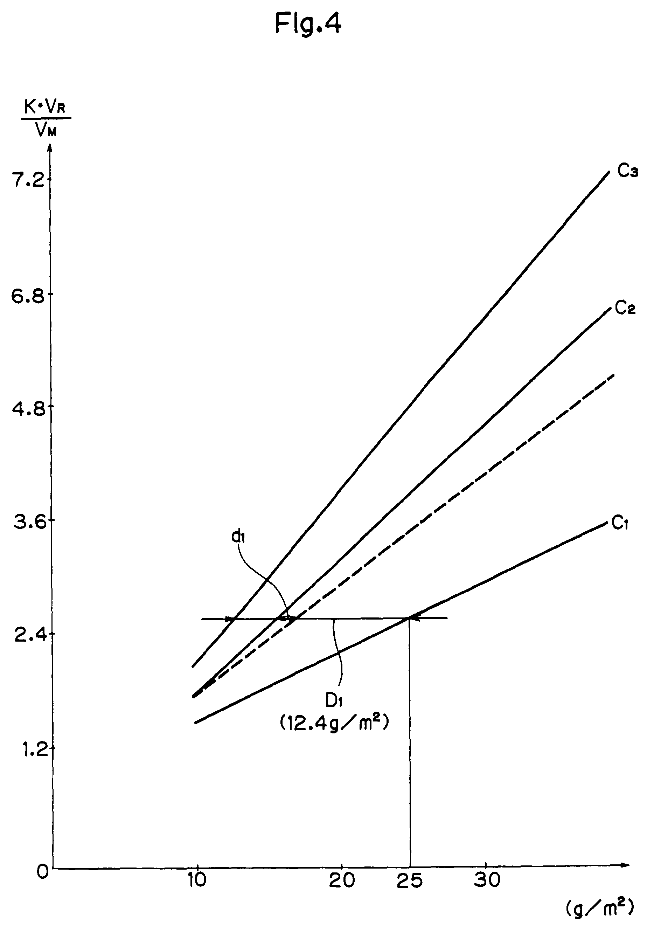

Fig. 4 shows calibration curves which are obtained using three kinds of samples in the conventional moisture meter which is shown in Fig. 2. In Fig. 4, the vertical axis designates the output of the moisture meter which is given by $K \cdot (V_R/V_M)$ (K: constant). The horizontal axis designates the moisture weight [g/m$^2$] of the paper. $C_1$ is a calibration curve when the basis weight of the paper is small, $C_2$ is a calibration curve when the basis weight of the paper is medium, and $C_3$ is a calibration curve when the basis weight of the paper is large.

For example, when the moisture weight is 25 [g/m$^2$], the output of the moisture meter according to the calibration curve $C_1$ is the smallest and the output of the moisture meter according to the calibration curve $C_3$ is the largest. The reason that the output according to the calibration curve $C_3$ is larger even the moisture weight is the same is supposed to be that, when the basis weight is large, the number of times of the reflection and scattering within the paper increases, the optical path length becomes substantially longer, the M rays are absorbed by moisture more, and the value of the output $V_M$ becomes smaller.

When the moisture weight is 25 [g/m$^2$], if the output of the moisture meter according to the calibration curve $C_1$ is regarded as the standard, there is a gap $D_1$ between the calibration curve $C_1$ and the calibration curve $C_3$ which gap $D_1$ is 12.5[g/m$^2$] if converted into moisture weight. If the gap is large, a gap $d_1$ between an object to be measured which is shown by a broken line and the calibration curve $C_2$ which is selected as the calibration curve, the characteristics of which are the nearest to that of the object, is large, which causes error of measurement. Further, the output according to the conventional system is shown as moisture weight, but generally, as a value according to which the quality of paper is to be controlled, moisture percentage is more convenient. Therefore, in addition to moisture weight, the basis weight of the paper is obtained to calculate moisture percentage.

Among the conventional infrared ray moisture meters which are referred to above, the one which is shown in Fig. 1 has advantages that the arrangement is simple and the attenuation of the quantity of rays is small, but on the other hand, as the object to be measured is only one point of the paper, there is a problem that, if the paper is thin, the moisture meter can not be sensitive. In the moisture meter which is shown in Fig. 2, as the optical axis of the light emitting portion and the optical axis of the light detecting portion are misaligned, the number of times of meeting of the rays with the paper is large. However, the range of the rays which are scattered at the paper is 180° at the widest, and rays which meets with the paper only once are included. Therefore, the sensitivity of the moisture meter is not satisfactory. Further, there is a problem that if the optical axis of the light emitting portion is shifted more from the optical axis of the light detecting portion in order to decrease the influence of rays which penetrate the paper only once, the quantity of rays is decreased (The conventional moisture meter is designed so that the optical axes of the light emitting portion and of the light detecting portion may be shifted from each other by about 60mm, and the distance between the upper reflector and the lower reflector may be about 6-8mm).

As for the moisture meter which is shown in Fig. 3, as rays which do not penetrate and are not scattered by the paper so many times (low-sensitive rays which do not meet with water molecules enough) are included in the rays to be detected, there is a problem that the sensitivity of moisture detection is low. Further, in this method, the sensitivity is different depending on whether the paper is thin or thick, and accordingly, there is a problem that the influence of the quality of paper is great.

Further, in the conventional moisture meters which are referred to above, if the axis of the head which contains the light emitting portion and the axis of the head which contains the light detecting portion are misaligned within a horizontal plane, a great error is caused, and accordingly, there is a problem that mechanical or electrical correcting means for correcting the error is necessary.

The present invention is made to solve the problems of conventional moisture meters which are referred to above, and the object of the present invention is to provide a device for measuring characteristics of paper and an infrared ray moisture meter in which transmission and scattering by paper is done enough independent of whether the paper is thin or thick, the sensitivity of which is high, and in which attenuation of rays is small and error of measurement with respect to misalignment in a horizontal plane is small, and to provide an infrared ray moisture meter in which the influence of the quality of paper is lowered, the shift of calibration curves is small, and moisture percentage is directly output.

## SUMMARY OF THE INVENTION

According to the present invention there is provided a device device for measuring characteristics of paper, comprising a first reflector, a second reflector being disposed opposite said first reflector with a space therebetween for paper to be examined to be movable therethrough, means for allowing light to enter the space between the reflectors so as to pass through the paper to be examined, and detecting means for detecting light passed through the paper to be examined for measuring physical characteristics of the paper according to a signal from the detecting means, characterized in that said first reflector has a first return portion provided at a periphery thereof for returning light, said second reflector has a second return portion provided at a periphery thereof for returning light, a shielding plate is disposed between one of said reflectors and the paper, said shielding plate having two mirror finished sides and comprising means for guiding light that has passed through the paper to be examined to the detecting means, wherein said first and second return portions of the first and second reflectors reflecting the light within the space reaching the periphery of the reflectors to return towards the shielding plate.

Preferred embodiments of the device for measuring characteristics of paper are defined in the dependent claims.

According to the present invention there is also provided an infrared ray moisture meter for measuring a moisture content of paper, characterized by comprising such a device for measuring characteristics of paper according to the invention, means for applying to said means for allowing light to enter the space between the reflectors so as to pass through the paper to be examined infrared light of a first wavelength which is absorbed by moisture, infrared light of a second wavelength which is absorbed by cellulose, and infrared light of a third wavelength which is absorbed by neither moisture nor cellulose, said detecting means for detecting light passed through the paper to be examined is adapted to detect the light of the above wavelengths and to generate output signals corresponding thereto, respectively, and calculating means for calculating the moisture content of the paper from the output signals of the detecting means.

BRIEF DESCRIPTION OF THE DRAWING

Figs. 1 through 3 are views which show conventional moisture meters;

Fig. 4 is a graph of characteristics of calibration curves which are obtained by the conventional moisture meter that is shown in Fig. 2;

Fig. 5 is a sectional perspective view which shows a device for measuring characteristics of paper according to the invention for use in an infrared ray moisture meter as an embodiment of the present invention;

Fig. 6 is a graph which shows the relationship between a signal of measurement ($V_R/V_M$), MW, and calibration curves in a conventional moisture meter;

Fig. 7 is a graph which shows the relationship between a signal of measurement ($V_R/V_M$), MW, and calibration curves in a moisture meter of the present invention;

Fig. 8 is a theoretical block diagram of an infrared ray moisture meter of the present invention which uses rays of three different wavelength ranges;

Fig. 9 is a graph of characteristics of calibration curves which are obtained by an infrared ray moisture meter of the present invention;

Fig. 10 is a comparative table of calibration curves which are obtained by an infrared ray moisture meter of the present invention which uses rays of three different wavelength ranges and calibration curves which are obtained by a conventional moisture meter;

Fig. 11 is a sectional view which shows an embodiment of the present invention wherein the outer diameter of an upper reflector is formed so as to be smaller than the outer diameter of a lower reflector;

Fig. 12 is a graph which shows the relationship of the amount of misalignment and error of moisture percentage when the outer diameter of the upper reflector is formed so as to be of the same size as the outer diameter of the lower reflector;

Fig. 13 is a graph which shows the relationship of the amount of misalignment and error of moisture percentage when the outer diameter of the upper reflector is formed so as to be smaller than the outer diameter of the lower reflector; and

Fig. 14 is a sectional perspective view which shows an embodiment of the present invention wherein the measurement width is made to be narrow.

DETAILED DESCRIPTION OF THE PREFERRED EMBODIMENTS

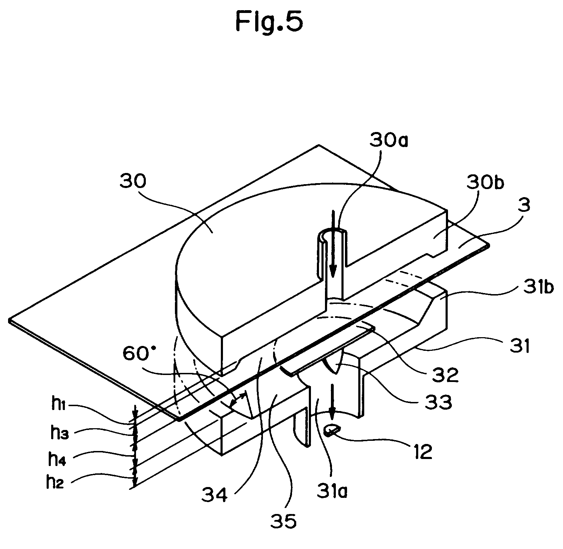

Fig. 5 is a partial sectional perspective view which shows an embodiment of the invention of a device for measuring characteristics of paper to be used in an infrared ray moisture meter of the present invention.

In Fig. 5, the side which faces paper 3 of an upper or first reflector 30 is mirror finished. A light emitting hole 30a is formed at the center of the upper reflector 30. A return ring 30b is formed at the peripheral portion of the upper reflector 30. The return ring 30b is convex and the section of its inner circumference face meets at about 60° to the perpendicular of the mirror finished face.

The side which faces the paper 3 of a lower or second reflector 31 is also mirror finished. A light detecting hole 31a is formed at the center of the lower reflector 31. A return ring 31b is formed at the peripheral portion of the lower reflector 31. The return ring 31b is convex and the section of its inner circumference face meets at about 60° to the perpendicular of the mirror finished face.

Both of the sides of a shielding plate 32 are mirror finished. A conical protrusion (a conical mirror) 33 the surface of which is mirror finished is provided at the center of one of the sides of the shielding plate 32. The shielding plate 32 is fixed by a plurality of supporting poles (not shown) in the space between the lower reflector 31 and the paper 3 so as to be as high as the upper portion of the return ring 31b of the lower reflector 31. The conical mirror 33 is disposed on the side of the light detecting hole 31a. The center of the shielding plate 32 is preferably aligned with the axis of the upper reflector 30 and the axis of the lower reflector 31.

The upper and lower reflectors 30 and 31 sandwich the paper 3 the moisture contained in which is to be measured. The upper and lower reflectors 30 and 31 are disposed so as to be as adjacent as possible to each other taking necessary allowable range into consideration and form light emitting space 34 and light detecting space 35. Although not shown in Fig. 5, a light source is disposed over the light emitting hole 30a which light source applies M rays the wavelength of which is 1.94μm and which is absorbed by moisture and R rays the wavelength of which is 1.8μm and which is not absorbed by moisture. Elements such as a calculating portion which conducts calculation of moisture based on output of a light detector 12 which are necessary for a moisture meter are provided at the subsequent stage of the light detector 12.

In the above arrangement, among rays which are applied from the light emitting portion to the surface of the paper 3, rays which are scattered at the surface of the paper 3 are reflected at the upper reflector 30, and rays which penetrate the paper 3 are reflected at the shielding plate 32 and are returned to the paper. In this way, rays which penetrate or are scattered at the paper 3 and which are conveyed around are mainly reflected at the reflecting surface of the upper reflector 30, are returned to the center portion, and repeat penetration of or scattering at the paper 3. A part of the rays is reflected between the shielding plate 32 and the lower reflector 31 and reach the conical mirror 33 under the shielding plate 32. The conical mirror 33 has a function of leading the rays effectively to the light detector 12.

The applicant of the present invention has conducted an experiment under the condition that the outer diameter of the upper and the lower reflectors is 60mm, the height h1 of the upper return ring 30b is 2.5mm, the height h2 of the lower return ring 31b is 5.0mm, the distance h3 from the upper return ring 30b to the paper and the distance h4 from the lower return ring 31b to the paper are 2.0mm, the diameter of the shielding plate 32 is 30mm, the distance from the surface of the shielding plate to the paper is 2.0mm, the diameter of the light emitting hole 30a is 3mm, the diameter of the light detecting hole 31a is 18mm, and all other conditions are the same as that of a conventional moisture meter.

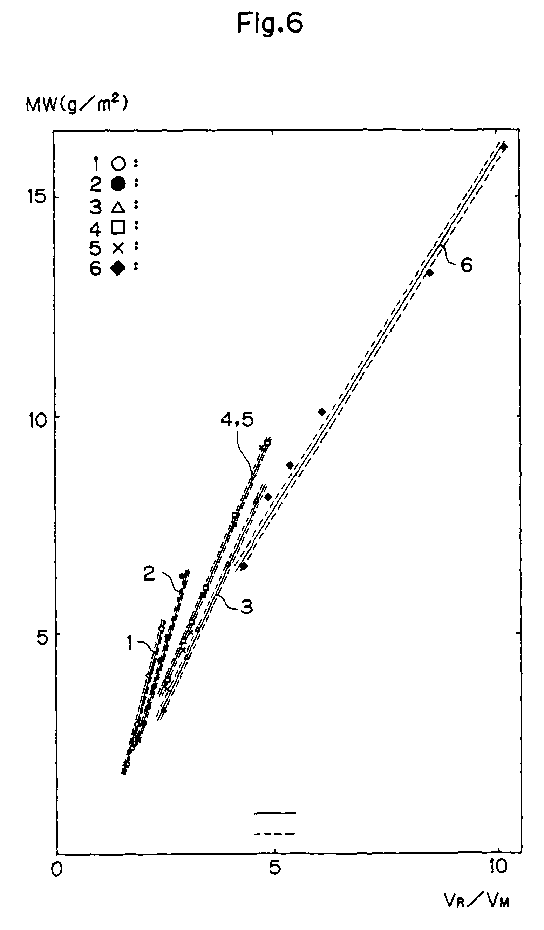

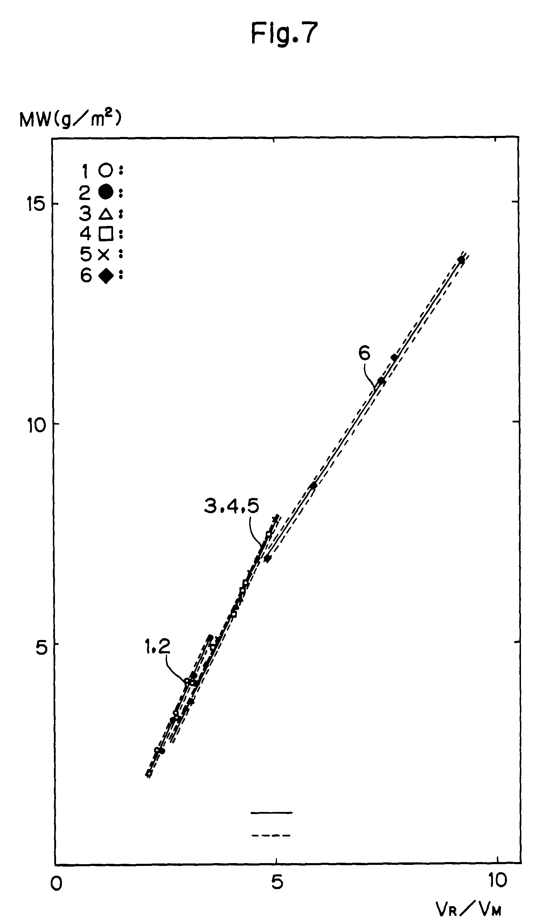

Figs. 6 and 7 show calibration curves of the conventional moisture meter which is shown in Fig. 2 and the moisture meter of the present invention which is referred to above when, with respect to six kinds of paper, MW (moisture weight per unit area) is found based on a signal of measurement ($V_R/V_M$) and the moisture percentage of the paper (MW/BW×100% ... BW; paper weight per unit area) is calculated based on the above value so that measurement may be conducted within an accuracy of ± 0.1% (an accuracy of ± 0.1% shall mean, for example, when the moisture percentage is 5%, the error of measurement is within the range of 4.9% - 5.1%). With the conventional moisture meter, as shown in Fig. 6, five calibration curves are necessary to obtain an accuracy of ± 0.1%. On the other hand, with the moisture meter of the present invention, as shown in Fig. 5, only three calibration curves are necessary.

According to the above arrangement, rays which come from the light emitting hole first penetrate or are scattered by the paper. These rays are reflected between the shielding plate and the upper reflector and meet with the paper a plurality of times. After the rays reach the periphery of the shielding plate, a part of the rays repeat diffraction between the lower reflector and the rear surface of the shielding plate and then reach the light detector. On the other hand, rays which repeat meeting with the paper further toward the periphery of the reflectors are returned by the return rings and again reach the periphery of the shielding plate, and a part of the rays are diffracted toward the rear surface of the shielding plate, repeat penetration and scattering, and reach the light detector. As a result, rays which are low in sensitivity of moisture detection, for example, rays which penetrate the paper only once, do not reach the light detector, which leads to an improvement of sensitivity.

Further, as the moisture meter is arranged to return rays to the side of the light detector (in the direction of the center) by the return rings, the confining effect is made to be high, the quantity of rays of detection is made to be large, and the same optical system can measure moisture of both paper of low basis weight (for example, about 30g/m$^2$) and paper of high basis weight (for example, about 150g/m$^2$).

Still further, as rays which meet with enough water molecules and which penetrate and are scattered by paper enough are detected independent of whether the paper is thin or thick, the influence of the quality of the paper is lowered.

Also, as the rays are returned by the return rings, only a small area of paper to be measured is necessary in order to obtain rays of the same optical path length as that of a conventional meter.

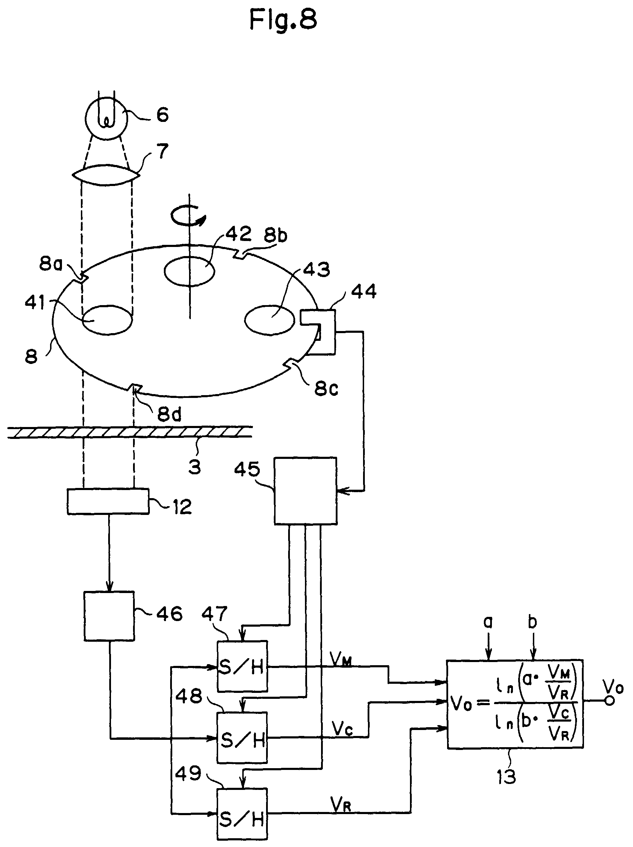

Fig. 8 is a theoretical block diagram of an infrared ray moisture meter with which the influence of the quality of paper is lowered, the shift of the calibration curves is made to be small, and moisture percentage can directly be outputted.

In Fig. 8, a numeral 6 designates a lamp and a numeral 7 designates a condenser lens. Filters 41, 42, and 43 each of which selectively transmits infrared rays of a predetermined wavelength range are disposed on a filter wheel 8 so as to be disposed on a concentric circle of the filter wheel 8. Four cut-outs 8a, 8b, 8c, and 8d for generating a synchronisation signal are provided at the periphery of the filter wheel 8. A photo interruptor 44 in which an LED and a phototransistor are disposed so as to face each other and so as to sandwich the peripheral portion of the filter wheel 8 generates a synchronisation pulse every time the cut-out 8a, 8b, 8c, or 8d passes the photo interruptor 44. A synchronisation signal circuit 45 generates a synchronisation signal based on a synchronisation pulse from the photo interruptor 44.

A numeral 3 designates paper to be measured, a numeral 12 designates a light detector, and a numeral 46 designates an amplifier. Sample-and-hold circuits 47, 48, and 49 convert an alternating current signal which is given by the light detector 12 to a direct current signal according to a synchronisation signal from the synchronisation signal circuit 45. An arithmetic circuit 13 calculates signals which are given by the sample-and-hold circuits 47, 48, and 49 and generates moisture me-

ter output $V_o$.

Next, the operation of the moisture meter as an embodiment of the present invention is described. According to the rotation of the filter wheel 8, infrared rays the wavelength of which is 1.94μm and which pass the filter 41, infrared rays the wavelength of which is 2.1μm and which pass the filter 42, and infrared rays the wavelength of which is 1.8μm and which pass the filter 43 are applied by turns to the paper 3. Penetrating rays and scattered rays based on these three kinds of infrared rays are detected by the light detector 12. Then, detection output $V_M$ with respect to the infrared rays the wavelength of which is 1.94μm, detection output $V_C$ with respect to the infrared rays the wavelength of which is 2.1μm, and detection output $V_R$ with respect to the infrared rays the wavelength of which is 1.8μm are given in a form of an alternating current signal to the sample-and-hold circuits 47, 48, and 49. By a synchronisation signal which is given to the circuits 47, 48, and 49 by the synchronisation signal circuit 45, the alternating current signal is converted to a direct current signal. Then, the output $V_M$, $V_C$, and $V_R$ is outputted from the sample-and-hold circuits 47, 48, and 49, respectively.

At the arithmetic circuit 13, optimum constants $\underline{a}$ and b are set and the following calculation is conducted:

$$V_o = \{\ell n(a \cdot V_M/V_R)\}/\{\ell n(b \cdot V_C/V_R) \qquad (1)$$

As a result, $V_o$ becomes a function of only the moisture weight (MW) and the cellulose weight (CW) (MW/CW), and one calibration curve is made independent of whether the basis weight is large or small and what kind of pulp the paper is made of.

In the equation (1), error of the moisture meter caused by change of optical path length according to the enlargement of the basis weight is lowered by dividing $V_M$ by $V_C$ which have the same kind of optical path length. The reason for dividing $V_M$ and $V_C$ by $V_R$ is to remove the influence of loss by scattering. Further, as the absorption signal of moisture is divided by the absorption signal of cellulose, a signal which is related to moisture percentage (moisture weight / cellulose weight) is outputted, and by conducting ash content correction, moisture percentage alone can be outputted.

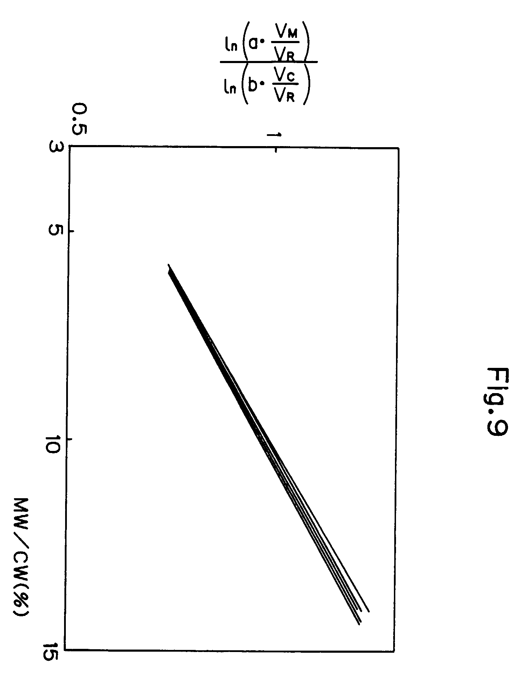

Fig. 9 shows calibration curves of seven specimens of newsprint using a system of the present invention (as some of the calibration curves lie on another, five calibration curves are shown). The vertical axis shows $V_o$ as referred to above, and the horizontal axis shows MW/CW [%].

The moisture percentage is expressed as follows:

$$1/\{1+(CW/MW) \cdot \{1/(1-A)\} \cdot 100[\%] \qquad (2)$$

A; ash percentage = ash weight / bone dry weight

Accordingly, if (MW/CW) is found, moisture percentage can be outputted alone.

In this case, ash content correction has to be conducted with respect to every paper. However, the influence of change of the ash percentage A of the equation (2) on the moisture percentage is small, and ash percentage which is found in advance using samples can be used.

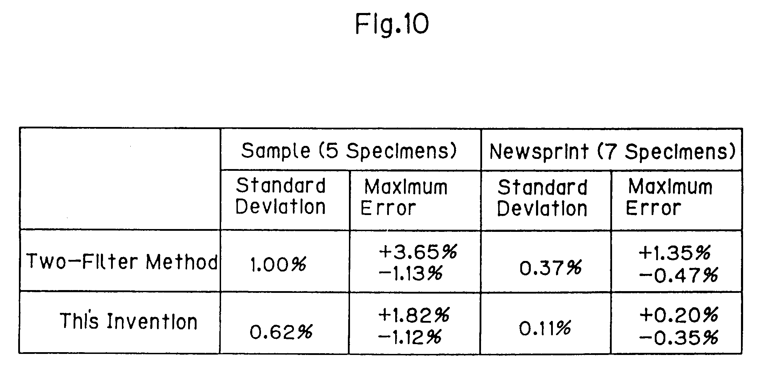

Fig. 10 shows comparison between calibration curves of a moisture meter as an embodiment of the present invention which uses rays of three different wavelength range and calibration curves of a conventional moisture meter of two-filter method. As is clear from Fig. 10, with respect to five specimens of sample, the standard deviation of error of moisture percentage is lowered from 1.00 to 0.62 [%]. Also, with respect to seven specimens of newsprint, the standard deviation of error of moisture percentage is lowered from 0.37 to 0.11 [%]. As for the maximum error of moisture percentage, with respect to the five specimens of sample, it is lowered from +3.65, -1.13 to +1.82, -1.12 [%]. With respect to the seven specimens of newsprint, the maximum error of moisture percentage is lowered from +1.35, -0.47 to +0.20, -0.35 [%].

Here, the standard deviation means standard deviation of one calibration curve which is obtained from calibration curves of each sample so that the error of moisture percentage may be the minimum (a moisture percentage error minimum calibration curve) and calibration curves of the sample (the range of moisture percentage is from 2% to 12%). The maximum error is the maximum value of plus and minus of error of moisture percentage between the moisture percentage error minimum calibration curve and the calibration curves of the sample within the range of 2% and 12%.

In the moisture meter of the present invention, the infrared rays the wavelength of which is 1.94μm, the infrared rays the wavelength of which is 2.1μm, and the infrared rays the wavelength of which is 1.8μm are separated by the filter wheel 8 and then applied to the paper 3. However, white light may be applied to the paper 3 and infrared rays of each wavelength may be separated from the light after penetrating or being scattered by the paper 3.

Further, in the moisture meter of the present embodiment, $\ell n$ is used as the functions, $f$, but a function such as $l_{og}$ or $X-\{(X-1)^2/2\}-1$ of polynomial expansion may be used as long as the function outputs an approximate value of logarithm.

According to the present invention, an infrared ray moisture meter with which moisture of paper is measured with the influence of the quality of the paper lowered, with shift of the calibration curve small, and which can directly output moisture percentage can be materialized.

Next, a moisture meter of the present invention in which error when misalignment is caused between the upper reflector 30 disposed on the light emitting side and the lower reflector 31 disposed on the light detecting side of Fig. 5 is prevented is described

Generally, in an on-line measurement of moisture of paper, a light emitting portion and a light detecting portion are disposed on two arms, respectively, which arms are parallel to each other, paper moves between the arms, and further, in order to measure the whole area of the width of the paper, the light emitting portion and the light detecting portion move orthogonally with respect to the machine direction along the arms. Accordingly, the position of the upper reflector 30 disposed on the light emitting side and the position of the lower reflector 31 disposed on the light detecting side (see Fig. 1) are not always aligned with each other and misalignment, for example, in a horizontal direction or the vertical-direction is caused. As this misalignment is followed by error of measurement, conventionally, various kinds of electrical or mechanical correcting means are proposed. With the moisture meter of the present invention which is shown in Fig. 5, the same kind of error which is caused by the misalignment also can not be prevented from happening.

When the upper and lower reflectors 30 and 31 are horizontally misaligned, the confining effect of the rays is lowered and the conditions of reflection with the shielding plate 32 are changed, and thus the quantity of rays of detection is decreased and the sensitivity is lowered.

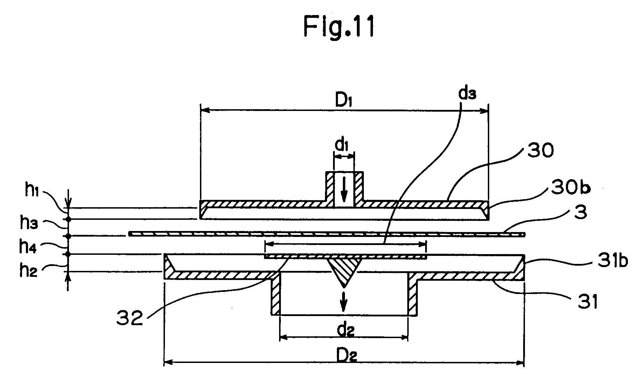

Fig. 11 is a partial sectional view of an embodiment with which error caused by misalignment of the light emitting portion and the light detecting portion is prevented from happening. In this embodiment, an improvement in the horizontal misalignment as referred to above is made. The outer diameter of the upper reflector 30 is made to be smaller than the outer diameter of the lower reflector 31. The lower reflector 31 is necessary for increasing the quantity of rays of detection. On the other hand, as detection of moisture is conducted on the area where the upper and lower surfaces are overlapped, the range of measuring moisture is the area where the upper reflector 30 exists.

According to the arrangement as referred to above, as far as the upper reflector 30 horizontally moves within the area over the lower reflector 31, the sensitivity of moisture detection and the quantity of rays are maintained at a predetermined level.

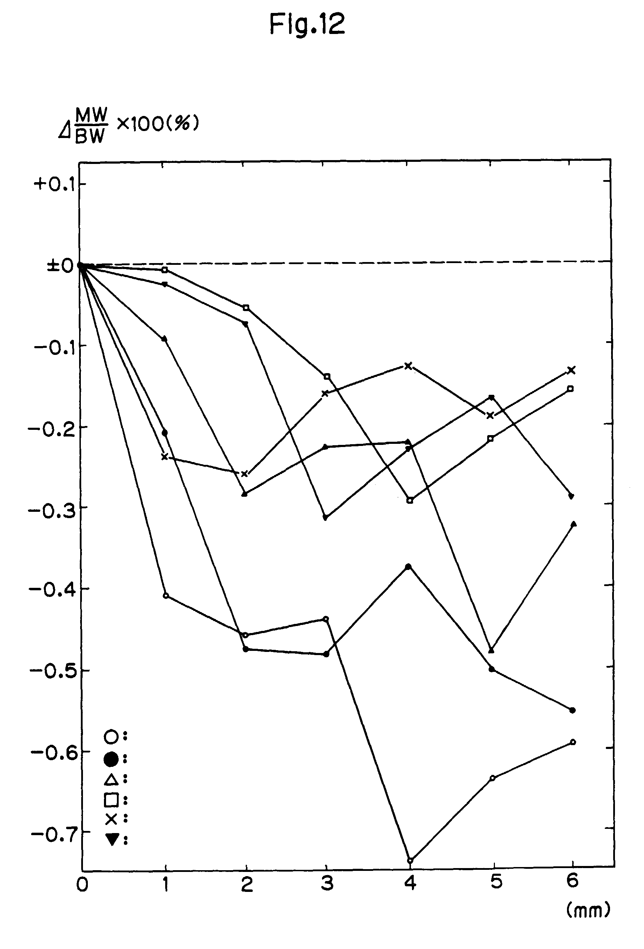

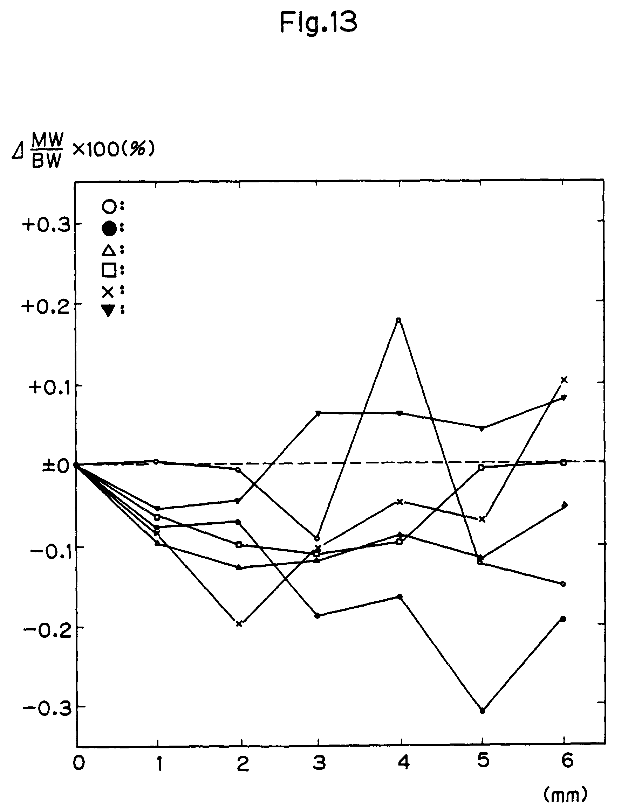

Figs. 12 and 13 show an example of an experiment using six kinds of paper of the relationship of the misalignment and error of moisture percentage in case the outer diameter of the upper reflector 30 is formed to be the same size as the outer diameter of the lower reflector 31 (Fig. 12) and in case the outer diameter of the upper reflector 30 is formed to be smaller than the outer diameter of the lower reflector 31 (Fig. 13) of the moisture meter using the rays of three different wavelength ranges of the present invention which is shown in Fig. 8. The results are the errors of moisture percentage from a standard moisture percentage when there is no alignment (the upper reflector 30 is coaxial with the lower reflector 31) of moisture percentage (MW/BWX100%) found by signals which are obtained by moving the upper head by every 1mm from the center with the lower head fixed.

As is clear from Figs. 12 and 13, by changing the diameter of the upper reflector 30 from the diameter of the lower reflector 31, the movement characteristics can be greatly improved (generally, the quantity of misalignment between the upper and the lower heads is about $\pm$ 1.5mm, but in Fig. 12, with respect to tracing paper-1 and tracing paper-2, the error as to the misalignment of 2mm is over 0.2%. On the other hand, in Fig. 13, when the misalignment is 2mm, the error is within 0.2% with respect to every kind of paper, and the error of moisture percentage is improved).

In the experiment, a moisture meter in which the outer diameters $D_1$ and $D_2$ of the upper and the lower reflectors 30 and 31 are both 40mm and a moisture meter in which the outer diameter $D_1$ of the upper reflector 30 is 40mm and the outer diameter $D_2$ of the lower reflector 31 is 50mm are used. The diameter $d_1$ of the light emitting hole, the diameter $d_2$ of the light detecting hole, and the diameter $d_3$ of the shielding plate are commonly 3mm, 18mm, and 22mm, respectively. The height $h_1$ of the return ring 30a is 2.5mm, the height $h_2$ of the return ring 30b is 5.0mm, and the distances $h_3$ and $h_4$ from the return rings 30a and 30b to the paper are both 2.0mm.

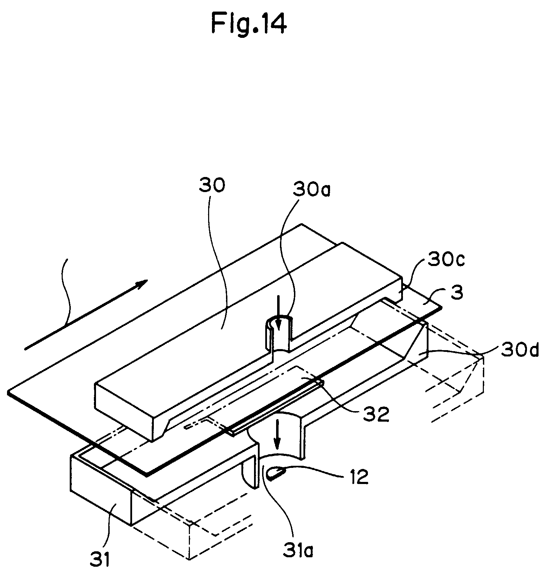

Fig. 14 is a sectional perspective view which shows an embodiment of the present invention in which the measurement width is made to be smaller without lowering the sensitivity of detection. Like reference characters in Figs. 5 and 14 designate like elements and duplicated description is omitted. In the moisture meter of the present embodiment, the upper and the lower reflectors 30 and 31 are formed to be rectangular. The longer side of the upper reflector 30 is 140mm, the shorter side of the upper reflector 30 is 40mm, the longer side of the lower reflector 31 is 150mm, and the shorter side of the lower reflector 31 is 50mm. The height $h_1$ and $h_2$ of the return rings 30b and 31b is 9.6mm from the reflection face. The diameter of the light emitting hole 30a is 3mm and the diameter of the light detecting hole 31a is 18mm. In the moisture meter of the present embodiment, the shielding plate 32 is square-like one side of which square is about 40mm, both of which sides in the longitudinal direction of the lower reflector 31 are bent so as to be broadened toward the ends, the bent portions being in contact with the lower reflector and being fixed around the center of the lower reflector so that opened portions are located in the machine direction. This device is fixed to a mounting head with its longitudinal direction being along the machine direction.

According to the arrangement as referred to above, the penetrating and scattered rays do not come from the cross direction and only rays which penetrate and are scattered along the openings in the machine direction are detected. Generally, in an on-line measurement of moisture of paper, the measurement width in the cross direction is more important than the measurement width in the machine direction. This arrangement is short in the cross direction and long in the machine direction, and therefore, the measurement width can be shortened without lowering the sensitivity of detection. In the embodiment, the upper and lower reflectors are rectangular, but they may be formed to be ellipse-like the longer sides of which are straight and the shorter sides of which are semicircular.

## Claims

1. A device for measuring characteristics of paper, comprising

   a first reflector (30);
   a second reflector (31) being disposed opposite said first reflector (30) with a space (34) therebetween for paper (3) to be examined to be movable therethrough;
   means (30a) for allowing light to enter the space (34) between the reflectors (30,31) so as to pass through the paper (3) to be examined; and
   detecting means (12) for detecting light passed through the paper (3) to be examined for measuring physical characteristics of the paper (3) according to a signal from the detecting means (12);
   characterized in that
   said first reflector (30) has a first return portion (30b,30c) provided at a periphery thereof for returning light;
   said second reflector (31) has a second return portion (31b,30d) provided at a periphery thereof for returning light;
   a shielding plate (32) is disposed between one of said reflectors (30,31) and the paper (3), said shielding plate (32) having two mirror finished sides and comprising means (33) for guiding light that has passed through the paper (3) to be examined to the detecting means (12);
   wherein said first and second return portions (30b,30c,31b,30d) of the first and second reflectors (30,31) reflecting the light within the space (34) reaching the periphery of the reflectors (30,31) to return towards the shielding plate (32).

2. The device of claim 1, characterized in that the first and the second reflector (30,31) are disc shaped and have substantially the same outer diameters.

3. The device of claim 1, characterized in that the first and the second reflector (30,31) are disc shaped and have different outer diameters.

4. The device of claim 3, characterized in that the outer diameter of the first reflector (30) is smaller than the outer diameter of the second reflector (31).

5. The device of claim 1, characterized in that the first and the second reflector (30,31) are rectangular in shape with the longer dimensions of each reflector being different from each other.

6. The device of claim 5, characterized in that the longer sides of the first and the second reflector (30,31) are disposed in the moving direction of the paper.

7. The device of claim 1, characterized in that the first and the second reflector (30,31) are formed to be ellipse-like.

8. The device of any one of claims 1 to 7, characterized in that first and the second return portions (30b, 30c,31b,30d) provided at the periphery of the reflectors (30,31) are formed as convex rings with a section of their inner circumference surface inclined at about 60° with respect to a line perpendicular to the surfaces of the reflectors (30,31).

9. An infrared ray moisture meter for measuring a moisture content of paper, characterized by comprising

   a device for measuring characteristics of paper according to any one of claims 1 to 8;
   means for applying to said means (30a) for allowing light to enter the space (34) between the reflectors (30,31) so as to pass through the paper (3) to be examined infrared light of a first wavelength which is absorbed by moisture, infrared light of a second wavelength which is absorbed by cellulose, and infrared light of a third wavelength which is absorbed by neither moisture nor cellulose;
   said detecting means (12) for detecting light passed through the paper (3) to be examined is adapted to detect the light of the above wavelengths and to generate output signals ($V_M$,$V_C$, $V_R$) corresponding thereto, respectively; and calculating means (13) for calculating the moisture content of the paper from the output signals ($V_M$,$V_C$,$V_R$) of the detecting means (12).

## Patentansprüche

1. Vorrichtung zum Messen oder Bestimmen der Eigenschaften von Papier, umfassend:

   einen ersten Reflektor (30),
   einen zweiten Reflektor (31), der dem ersten Reflektor (30) mit einem dazwischen gebildeten Zwischenraum (34) für Hindurchbewegung des zu untersuchenden Papiers (3) gegenüberstehend angeordnet ist,
ein Mittel (30a) zur Ermöglichung eines Eintritts von Licht in den Zwischenraum (34) zwischen den Reflektoren (30, 31), um das Licht das zu untersuchende Papier (3) durchdringen zu lassen, und
ein Detektionsmittel (12) zum Detektieren bzw. Erfassen des durch das zu untersuchende Papier (3) hindurchgedrungenen Lichts zwecks Messung physikalischer Eigenschaften des Papiers (3) entsprechend einem Signal vom Detektionsmittel (12),
dadurch gekennzeichnet, daß
der erste Reflektor (30) einen an seinem Umfang vorgesehenen ersten Rückstrahlabschnitt (return portion) (30b, 30c) zum Rückstrahlen oder Zurückleiten von Licht aufweist,
der zweite Reflektor (31) einen an seinem Umfang vorgesehenen zweiten Rückstrahlabschnitt (31b, 30d) zum Rückstrahlen von Licht aufweist, (und)
zwischen einem der Reflektoren (30, 31) und dem Papier (3) eine Abschirmplatte (32) angeordnet ist, die zwei Spiegelschliff-Seiten und ein Mittel (33) zum Leiten von Licht, welches das zu untersuchende Papier (3) durchdrungen hat, zum Detektionsmittel (12) aufweist,
wobei die ersten und zweiten Rückstrahlabschnitte (30b, 30c, 31b, 30d) der ersten und zweiten Reflektoren (30, 31) das im Zwischenraum (34) den Umfang der Reflektoren (30, 31) erreichende Licht reflektieren, um es zur Abschirmplatte (32) zurückzuwerfen.

2. Vorrichtung nach Anspuch 1, dadurch gekennzeichnet, daß erster und zweiter Reflektor (30, 31) scheibenförmig sind und praktisch gleiche Außendurchmesser besitzen.

3. Vorrichtung nach Anspruch 1, dadurch gekennzeichnet, daß erster und zweiter Reflektor (30, 31) scheibenförmig sind und unterschiedliche Außendurchmesser besitzen.

4. Vorrichtung nach Anspruch 3, dadurch gekennzeichnet, daß der Außendurchmesser des ersten Reflektors (30) kleiner ist als der Außendurchmesser des zweiten Reflektors (31).

5. Vorrichtung nach Anspruch 1, dadurch gekennzeichnet, daß erster und zweiter Reflektor (30, 31) jeweils rechteckig geformt sind, wobei die längeren Abmessungen der jeweiligen Reflektoren voneinander verschieden sind.

6. Vorrichtung nach Anspruch 5, dadurch gekennzeichnet, daß die längeren Seiten von erstem und zweiten Reflektor (30, 31) in der Bewegungsrichtung des Papiers angeordnet sind.

7. Vorrichtung nach Anspruch 1, dadurch gekennzeichnet, daß erster und zweiter Reflektor (30, 31) elliptisch geformt sind.

8. Vorrichtung nach einem der Ansprüche 1 bis 7, dadurch gekennzeichnet, daß die am Umfang der Reflektoren (30, 31) vorgesehenen ersten und zweiten Rückstrahlabschnitte (30b, 30c, 31b, 30d) als konvexe Ringe geformt sind, wobei ein(e) Schnitt(linie) (section) ihrer Innenumfangsfläche unter etwa 60° zu einer senkrecht zu den Oberflächen der Reflektoren (30, 31) stehenden Linie geneigt ist.

9. Infrarot-Feuchtigkeitsmeßgerät zum Messen eines Feuchtigkeitsgehalts von Papier, gekennzeichnet durch

eine Vorrichtung zum Messen oder Bestimmen der Eigenschaften von Papier nach einem der Ansprüche 1 bis 8,
Mittel zum Einstrahlen (applying) von Infrarotlicht einer ersten Wellenlänge, das von Feuchtigkeit absorbiert wird, Infrarotlicht einer zweiten Wellenlänge, das von Zellulose absorbiert wird, und Infrarotlicht einer dritten Wellenlänge, das weder von Feuchtigkeit noch von Zellulose absorbiert wird, in das Mittel (30a) zur Ermöglichung eines Eintritts von Licht in den Zwischenraum (34) zwischen den Reflektoren (30, 31), um das Licht das zu untersuchende Papier (3) durchdringen zu lassen,
wobei das Detektionsmittel (12) zum Detektieren des durch das zu untersuchende Papier (3) hindurchgedrungenen Lichts das Licht der oben angegebenen Wellenlängen zu detektieren bzw. zu erfassen und jeweils entsprechende Ausgangssignale ($V_M$, $V_C$, $V_R$) zu erzeugen vermag, und
eine Recheneinheit (13) zum Berechnen des Feuchtigkeitsgehalts des Papiers anhand der Ausgangssignale ($V_M$, $V_C$, $V_R$) des Detektionsmittels (12).

## Revendications

1. Dispositif pour mesurer les caractéristiques du papier, comprenant:

- un premier réflecteur (30),
- un second réflecteur (31) disposé en face dudit premier réflecteur (30) avec un espace vide (34) entre eux à travers lequel peut être déplacé un papier (3) que l'on veut examiner,
- un moyen (30a) pour permettre à la lumière de pénétrer dans l'espace (34) entre les réflecteurs (30, 31) de façon à traverser le papier (3) que l'on veut examiner, et

- un moyen de détection (12) pour détecter la lumière qui a traversé le papier (3) que l'on veut examiner afin de mesurer des caractéristiques physiques du papier (3) d'après un signal fourni par le moyen de détection (12),
caractérisé en ce que :

- ledit premier réflecteur (30) comporte en sa périphérie une première partie de renvoi (30b, 30c) qui renvoie la lumière,

- ledit second réflecteur (31) comporte en sa périphérie une seconde partie de renvoi (31b, 30d) qui renvoie la lumière,

- une plaque formant écran (32) est disposée entre l'un desdits réflecteurs (30, 31) et le papier (3), ladite plaque formant écran (32) comportant deux faces à fini miroir et comprenant un moyen (33) pour guider vers le moyen de détection (12) la lumière qui a traversé le papier (3) que l'on veut examiner ,
dans lequel lesdites premières et secondes parties de renvoi (30b, 30c, 31b, 30d) des premier et second réflecteurs (30, 31) réfléchissent la lumière dans l'espace (34) qui atteint la périphérie des réflecteurs (30, 31) pour la renvoyer vers la plaque formant écran (32).

2. Dispositif selon la revendication 1, caractérisé en ce que les premier et second réflecteurs (30, 31) sont en forme de disques et ont sensiblement le même diamètre extérieur.

3. Dispositif selon la revendication 1, caractérisé en ce que les premier et second réflecteurs (30, 31) sont en forme de disques et ont des diamètres extérieurs différents.

4. Dispositif selon la revendication 3, caractérisé en ce que le diamètre extérieur du premier réflecteur (30) est plus petit que le diamètre extérieur du second réflecteur (31).

5. Dispositif selon la revendication 1, caractérisé en ce que les premier et second réflecteurs (30, 31) sont de forme rectangulaire, les longueurs des réflecteurs étant différentes l'une de l'autre.

6. Dispositif selon la revendication 5, caractérisé en ce que les longueurs des premier et second réflecteurs (30, 31) sont placées dans la direction de déplacement du papier.

7. Dispositif selon la revendication 1, caractérisé en ce que les premier et second réflecteurs (30, 31) sont de forme elliptique.

8. Dispositif selon l'une quelconque des revendications 1 à 7, caractérisé en ce que les premières et secondes parties de renvoi (30b, 30c, 31b, 30d) placées à la périphérie des réflecteurs (30, 31) sont formées comme des anneaux convexes dont une section de la surface périphérique intérieure est inclinée d'environ 60° par rapport à une ligne perpendiculaire aux surfaces des réflecteurs (30, 31).

9. Appareil de mesure de l'humidité par infrarouges servant à mesurer la teneur en humidité d'un papier, caractérisé en ce qu'il comprend :

- un dispositif servant à mesurer les caractéristiques du papier, conforme à l'une quelconque des revendications 1 à 8,

- un moyen pour appliquer, audit moyen (30a) qui permet à la lumière de pénétrer dans l'espace (34) entre les réflecteurs (30, 31) afin qu'elle traverse le papier (3) que l'on veut examiner, une lumière infrarouge ayant une première longueur d'onde qui est absorbée par l'humidité, une lumière infrarouge ayant une seconde longueur d'onde qui est absorbée par la cellulose et une lumière infrarouge ayant une troisième longueur d'onde qui n'est absorbée ni par l'humidité ni par la cellulose,

- ledit moyen de détection (12) qui détecte la lumière ayant traversé le papier (3) que l'on veut examiner est capable de détecter la lumière ayant les longueurs d'onde ci-dessus et de produire des signaux de sortie ($V_M$, $V_C$, $V_R$) qui correspondent respectivement à ces dernières, et

- un moyen de calcul (13) qui calcule la teneur en humidité du papier à partir des signaux de sortie ($V_M$, $V_C$, $V_R$) du moyen de détection (12).

# Fig.1

# Fig.2

# Fig.3

# Fig.4

# Fig.5

EP 0 453 797 B1

# Fig.6

## Fig.7

# Fig.8

$$V_0 = \frac{l_n\left(a \cdot \frac{V_M}{V_R}\right)}{l_n\left(b \cdot \frac{V_C}{V_R}\right)}$$

Fig.9

## Fig.10

| | Sample (5 Specimens) | | Newsprint (7 Specimens) | |
|---|---|---|---|---|
| | Standard Deviation | Maximum Error | Standard Deviation | Maximum Error |
| Two-Filter Method | 1.00% | +3.65%<br>−1.13% | 0.37% | +1.35%<br>−0.47% |
| This Invention | 0.62% | +1.82%<br>−1.12% | 0.11% | +0.20%<br>−0.35% |

EP 0 453 797 B1

Fig.11

EP 0 453 797 B1

# Fig.12

# Fig.13

# Fig.14